# EUROPEAN PATENT SPECIFICATION

(11) **EP 2 019 697 B1**
(45) Date of publication and mention of the grant of the patent: **04.01.2012**
(21) Application number: 07776581.6
(22) Date of filing: 30.04.2007
(51) Int. Cl.: A61L 31/02, A61L 31/10, A61L 31/16

(54) **NON-STICKY COATINGS WITH THERAPEUTIC AGENTS FOR MEDICAL DEVICES**
NICHTHAFTENDE BESCHICHTUNGEN MIT THERAPEUTISCHEN WIRKSTOFFEN FÜR MEDIZINISCHE VORRICHTUNGEN
REVÊTEMENTS NON COLLANTS AVEC AGENTS THÉRAPEUTIQUES POUR APPAREILS MÉDICAUX

(30) Priority: 01.05.2006 US 416488
(43) Date of publication of application: 04.02.2009
(73) Proprietor: Boston Scientific Limited, Christ Church (BB)
(72) Inventor: ZHOU, Pu, Maple Grove, MN 55311 (US)
(74) Representative: Hauck Patent- und Rechtsanwälte
(86) International application number: PCT/US2007/010567
(87) International publication number: WO 2007/130422

(56) References cited:
- EP-A- 1 479 401
- WO-A-02/26139
- WO-A-98/56312
- WO-A-2006/052574
- US-A1- 2004 059 409
- US-A1- 2006 088 654

## Description

### FIELD OF THE INVENTION

The invention relates generally to a medical device such as an intravascular stent that is useful for delivering a therapeutic agent to the body tissue of a patient, and a method for making such a device. More particularly, in one embodiment the invention is directed to a stent having a sidewall structure comprising a plurality of struts, in which the inner and outer surfaces of the struts are coated with different coating compositions.

### BACKGROUND OF THE INVENTION

Medical devices, such as implanted stents, have been coated with compositions comprising a therapeutic agent. One method of applying coatings loaded with a therapeutic agent to stents and other medical devices having a tubular portion is to coat the inside (adluminal surface), sides, and outside (abluminal surface) of the tubular portion of the medical device with the composition to form a continuous coating on the tubular portion. A reason for coating all these surfaces of the tubular portion of the medical device with a coating is to ensure adherence of the applied coating to the tubular portion. For example, when the tubular portion is comprised of struts, applying a coating composition to all surfaces of the struts will form a coating that wraps around the struts. The fact that the coating "wraps around" the struts enhances adherence of the coating to the tubular portion.

However, in many stents all of the surfaces of the medical device or portions thereof do not need to be coated with a coating composition comprising a therapeutic agent. For instance, in a vascular stent, the inner surface and side surfaces of the tabular portion may not have to be coated with a coating composition containing a therapeutic agent. This is because these parts of the stent do not come in direct contact with the body lumen wall and do not apply the therapeutic agent to the body lumen wall. Therefore, it is not necessary to coat the inner surface and sides of the stent struts with a coating composition containing a therapeutic agent that is being applied to the body lumen wall.

Moreover, in order to deliver certain stents, such as a balloon expandable stent, comprising a sidewall having struts, the stent must be put in its unexpanded state or "crimped" before it is delivered to a body lumen. Crimping can cause the coating composition to be torn or ripped off the struts. Specifically, if the first coating composition that is applied to the side surfaces of the struts of the stent contains a polymer that is relatively soft or tacky, then the coating composition will have a tendency to adhere to the side surfaces of adjacent struts during the crimping process. Such adherence will cause the coating composition to be ripped off the surfaces when the stent is expanded. Also, if the coating composition that is applied to the inner surface of the struts, which contacts the balloon, is coated with a material that is relatively soft or tacky, such coating will tend to be ripped off the inner surface because the coating will stick to the balloon as it contacts the inner surface during expansion. Therefore, there are problems associated with using relatively soft polymers in coatings. However, to form a coating containing a therapeutic agent, it is desirable to use such relatively soft polymer because such materials have a better ability to incorporate the therapeutic agent.

US 2004/0059409A1 discloses a method for applying coatings to a medical device. The outer surface and the inner surface of the medical device are covered by a first coating and a second coating, respectively. First and second coating form a continuous coating disposed on the medical device.

EP 1 479 401 A2 refers to drug-release coating. The coating is applied to the stent in such a way, that the entire surface is covered by the coating of a stent.

US 2006/0088654 A1 refers to a drug-release coated stent in which a second polymer composition is provided to an open lattice side wall stent.

WO 2006/052574 A2 refers to a stent for delivering a therapeutic agent having increased body tissue contact surfaces. The document is comprised in the state of art relevant to the question of novelty, pursuant to Art. 54(3), (4) EPC 1973. The document refers to a stent in which the side surfaces of the strut converge to one another in the direction of the outer surface in order to increase the body tissue contact surface of the stent.

Accordingly, there is a need for more efficient methods of coating a stent having a side wall comprised of struts, that can more accurately deliver the desired dosage of a therapeutic agent from the coating of the device in order to limit patient exposure to excess drug in the coating. Furthermore, there is a need for a coated expandable stent comprising struts in which the undesired removal of coating from the stent is minimized;

### SUMMARY OF THE INVENTION

These and other objectives are accomplished by the stent according to claim 1. In one embodiment, the invention is directed to an intravascular balloon- expandable stent, comprising a metal stent sidewall structure designed for implantation into a blood vessel of a patient. The sidewall structure comprises a plurality of openings therein and struts each having an outer surface (abluminal surface) and an inner surface (adluminal surface) opposite the outer surface.

There is a first coating composition disposed on the outer surface, inner surface and side surfaces of the struts, wherein the first coating composition comprises a therapeutic agent and a first biostable polymer. There is a second coating composition disposed on the first coating composition that is disposed on the inner surface and side surfaces of the struts, wherein the second coating composition comprises a second biostable polymer that has less tackiness than the first polymer and is free of any therapeutic agent. The second coating composition is not disposed on the first coating composition that is disposed on the outer surface of the struts.

In some embodiments, the therapeutic agent comprises an anti-thrombogenic agent, anti-angiogenesis agent, anti-proliferative agent, anti-restenosis agent, growth factor, radiochemical or antibiotic. In alternative embodiments, the therapeutic agent comprises paclitaxel, sirolimus, everolimus, tacrolimus, or pimecrolimus.

In other embodiments, the first polymer is biostable. Generally, the first polymer can comprise a styrene-isobutylene copolymer, polyurethane, silicone, polyester, polyolefin, polyisobutylene, ethylene-alphaolefin copolymer, acrylic polymer or copolymer, vinyl halide polymer, polyvinyl ether, polyvinylidene halide, polyacrylonitrile, polyvinyl ketone, polyvinyl aromatic, polyvinyl ester, copolymer of vinyl monomers, copolymer of vinyl monomers and olefins, polyamide, alkyd resin, polycarbonate, polyoxymethylene, polyimide, polyether, epoxy resin, polyurethane, rayon-triacetate, cellulose, cellulose acetate, cellulose butyrate, cellulose acetate butyrate, cellophane, cellulose nitrate, cellulose propionate, cellulose ethers, carboxymethyl cellulose, collagen, chitin, polylactic acid, polyglycolic acid, polylactic acid-polyethylene oxide copolymer, EPDM rubber, fluorosilicone, polyethylene glycol, polysaccharide, or phospholipid. In some embodiments, the stent sidewall structure is balloon expandable. In other embodiments, the sent sidewall structure comprises a metal.

In certain embodiments, the struts comprise at least one side surface adjacent to the outer surface and the inner surface and connects the inner surface and outer surface. The first coating composition is disposed on at least a portion of the side surface of at least some of the struts and the second coating composition is disposed on the first coating composition that is disposed on the side surface of the struts. In some embodiments, the first coating composition conforms to the outer surface and inner surface of the struts to preserve the openings of the stent sidewall structure and the second coating composition conforms to the inner surface of the at least one strut to preserve the openings of the sidewall structure.

The second polymer is harder than the first polymer. In other embodiments, the second polymer has a hardness of greater or more than about 40A. In particular embodiments, the second polymer has a tackiness of less than about 50g, such as about 3g to about 30g. In some embodiments, the first polymer has a tackiness of more than about 50g. Both of the first polymer and second polymer are biostable. In one embodiment, the polymer comprises a styrene-isobutylene copolymer, polyurethane, silicone, polyester, polyolefin, polyisobutylene, ethylene-alphaolefin copolymer, acrylic polymer or copolymer, vinyl halide polymer, polyvinyl ether, polyvinylidene halide, polyacrylonitrile, polyvinyl ketone, polyvinyl aromatic, polyvinyl ester, copolymer of vinyl monomers, copolymer of vinyl monomers and olefins, polyamide, alkyd resin, polycarbonate, polyoxymethylene, polyimide, polyether, epoxy resin, polyurethane, rayon-triacetate, cellulose, cellulose acetate, cellulose butyrate, cellulose acetate butyrate, cellophane, cellulose nitrate, cellulose propionate, cellulose ethers, carboxymethyl cellulose, collagen, chitin, polylactic acid, polyglycolic acid, polylactic acid-polyethylene oxide copolymer, EPDM rubber, fluorosilicone, polyethylene glycol, polysaccharide, or phospholipid.

In particular embodiments, the therapeutic agent comprises an anti-thrombogenic agent, anti-angiogenesis agent, anti-proliferative agent, anti-restenosis agent, growth factor, radiochemical or antibiotic. In other embodiments, the therapeutic agent comprises an anti-restenosis agent. More specifically, in certain embodiments, the therapeutic agent comprises paclitaxel, sirolimus, everolimus, tacrolimus, or pimecrolimus. In some embodiments, the stent sidewall structure is balloon-expandable. In other embodiments, the stent comprises a metal stent.

### BRIEF DESCRIPTION OF THE DRAWINGS

**Figure 1** depicts a perspective view of an implantable intravascular stent, having a sidewall comprising a plurality of struts with an outer surface, an inner surface, and side surfaces.

**Figure 1A** shows the outer surface, inner surface, and side surface of a strut of the implantable stent of **Figure 1****.**

**Figure 2** shows a cross-sectional view of an individual strut of a stent that has a first coating composition disposed on its outer surface.

**Figure 2A** shows a cross-sectional view of an individual strut of a stent that has a first coating composition disposed on its outer surface and side surfaces.

**Figure 3** is a cross-sectional view of a strut of a stent with a first coating composition disposed on the outer surface and inner surface and a second coating composition disposed on the first coating composition that is disposed on the inner surface.

**Figure 3A** is a cross-sectional view of a strut of a stent with a first coating composition disposed on the outer surface and inner surface and a second coating composition disposed on the side surfaces and the first coating composition that is disposed on the inner surface.

**Figure 3B** is a cross-sectional view of a strut of a stent with a first coating composition disposed on the outer surface, inner surface and side surfaces, and a second coating composition disposed on the first coating composition that is disposed on the inner surface.

**Figure 3C** is a cross-sectional view of a strut of a stent with a first coating composition disposed on the outer surface, inner surface and side surfaces, and a second coating composition disposed on the first coating composition that is disposed on the inner surface and side surfaces.

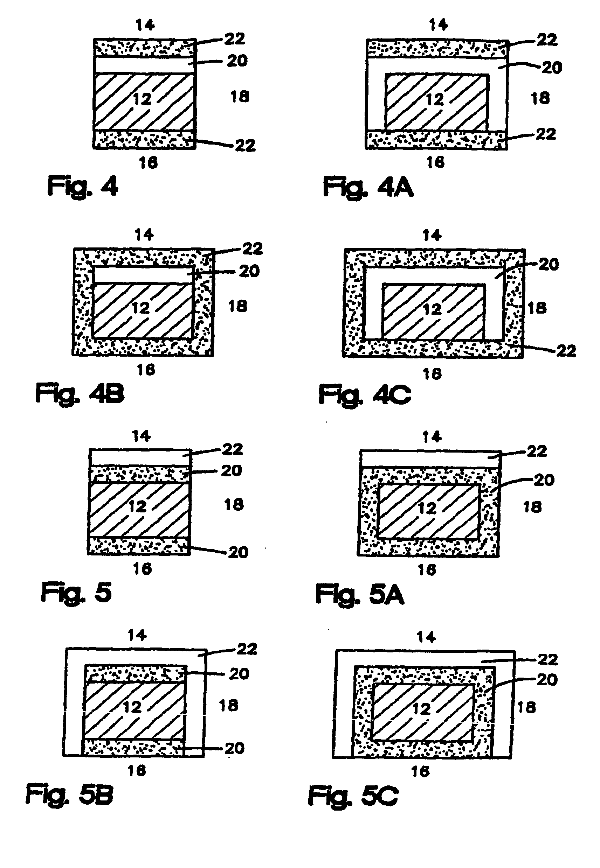

**Figure 4** is a cross-sectional view of a strut of a stent with a first coating composition disposed on the outer surface, and a second coating composition disposed on the inner surface and on the first coating composition that is disposed on the outer surface.

**Figure 4A** is a cross-sectional view of a strut of a stent with a first coating composition disposed on the outer surface and side surfaces, and a second coating composition disposed on the inner surface and on the first coating composition that is disposed on the outer surface.

**Figure 4B** is a cross-sectional view of a strut of a stent with a first coating composition disposed on the outer surface, and a second coating composition disposed on the inner surface and side surfaces, and on the first coating composition disposed on the outer surface.

**Figure 4C** is a cross-sectional view of a strut of a stent with a first coating composition disposed on the outer surface and side surfaces, and a second coating composition disposed on the inner surface, and on the first coating composition disposed on the outer surface and side surfaces.

**Figure 5** is a cross-sectional view of a strut of a stent with a first coating composition disposed on the outer and inner surfaces, and a second coating composition dispose on the first coating composition that is disposed on the outer surface.

**Figure 5A** is a cross-sectional view of a strut of a stent with a first coating composition disposed on the outer, inner and side surfaces, and a second coating composition disposed on the first coating composition that is disposed on the outer surface.

**Figure 5B** is a cross-sectional view of a strut of a stent with a first coating composition disposed on the outer and inner surfaces, and a second coating composition disposed on the side surfaces and first coating composition that is disposed on the outer surface.

**Figure 5C** is a cross-sectional view of a strut of a stent with a first coating composition disposed on the outer, inner and side surfaces, and a second coating composition disposed on the first coating composition that is disposed on the outer surface and side surfaces.

**Figure 6** shows an unexpanded stent disposed about a support.

### DESCRIPTION OF THE PREFERRED EMBODIMENTS

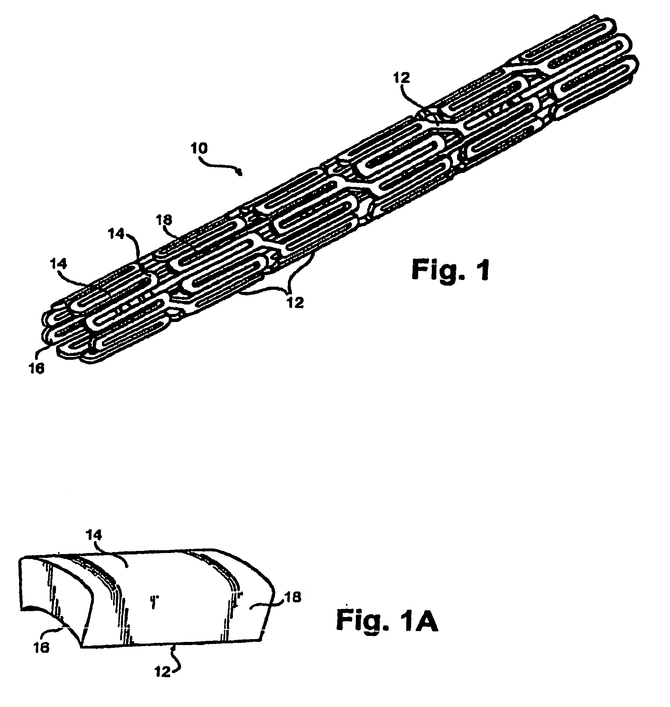

As shown in **Figure 1****,** the stents suitable for the present invention comprise a sidewall structure **10,** such as a tubular sidewall. Such a sidewall **10** is preferably comprised of a plurality of struts **12.** The struts **12** may be arranged in any suitable configuration. The struts **12** do not all have to have the same shape or geometric configuration. **Figure 1A** is a cross-sectional view of a stent strut **12** depicted in **Figure 1****.** Generally, each individual strut **12** has an outer surface or abluminal surface **14,** an inner surface or adluminal surface **16** opposite the outer surface **14,** and at least one side surface **18.** The outer surface **14** of the strut **12** is the surface that comes in direct contact with the body lumen wall when the stent is implanted. The outer surface **14** need not include only one flat surface or facet. Instead, it can be rounded, such as in the case of a wire strut **12,** or have a number of facets. The inner surface **16** of the strut **12** is the surface that is opposite the outer surface **14** and generally faces the interior of the lumen. The two side surfaces **18** are the surfaces of the strut **12** that are adjacent to the inner surface **16** or outer surface **14.** The side surface **18** connects the inner surface **16** and the outer surface **14.** Like the outer surface **14,** the inner surface **16** and side surface **18** can be rounded or have a number of facets.

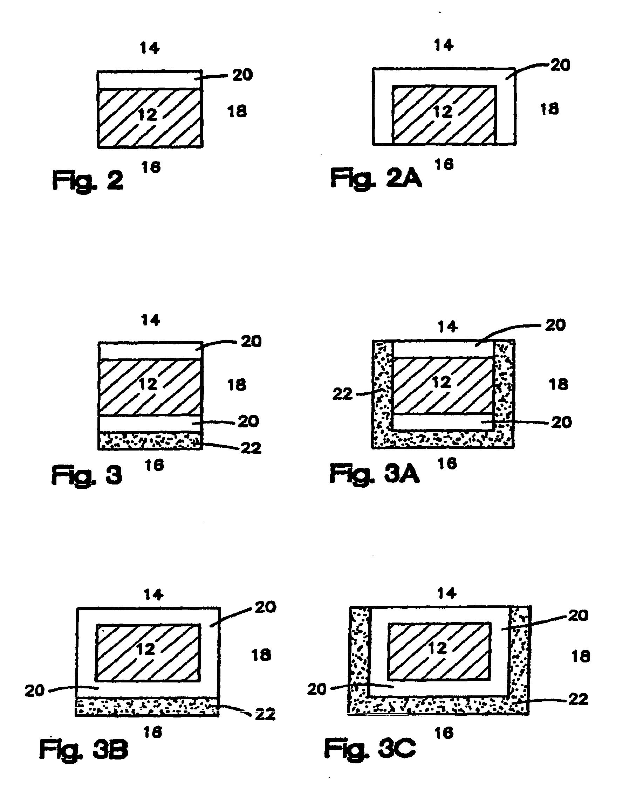

**Figure 3** is a cross-sectional view of an embodiment of a stent having a coating composition disposed thereon. As shown in **Figure 3****,** a first coating composition **20** is disposed on the outer surface **14** and inner surface **16** of the strut. The first coating composition **20** comprises a therapeutic agent and a first polymer. A second coating composition **22** is disposed on at least a portion of the first coating composition **20** that is disposed on the inner surface **16.** The second coating composition **22** is not disposed on the portion of the first coating composition **20** that is disposed on the outer surface **14.** The second coating composition **22** comprises a second polymer that has a less or different tackiness than the first polymer and is substantially free of the therapeutic agent, i.e. contains less than 1% by weight of the second coating composition. In some embodiments, the second coating composition is free of any therapeutic agent.

The tackiness of a material can be measured by a texture analyzer and is generally considered to be the force required to separate the probe of the texture analyzer from the test surface as it is lifted from the surface. The tackiness of a polymer can be measured by a texture analyzer when the compressive force is about 50 grams, when the compressive force time is about 5 seconds, when the upward test speed is about 25 mm/s and/or the downward test speed is 0.020 mm/s. In certain embodiments, the more tacky polymers should have a tackiness of 50 g or more, such as about 60g to about 80g, e.g. about 70g. The less tacky polymers should have a tackiness of 50 g or less such as about 3g to about 30g, e.g. about 16g.

The hardness of a polymer can be measured by a 3 spring-loaded indenter which assesses hardness by computing the resistance of a material to indentation. The higher the number reported, the greater the resistance. The ASTM test method for hardness is ASTM D2240. The ISO test method for hardness is ISO 868. In some embodiments, it may be preferable that the less tacky polymer have a hardness of greater than about 40A.

Another embodiment is shown in **Figure 3A** which is similar to the embodiment in **Figure 3****.** In this embodiment, the second coating composition **22** is also disposed on the side surfaces **18** of the strut.

As shown in **Figure 38,** in another embodiment that is similar to the embodiment in **Figure 3****,** the first coating composition **20** is also disposed on the side surfaces **18.**

The embodiment shown in **Figure 3C** is similar to the one shown in **Figure 3****.** However, in this embodiment, the first coating composition **20** is also disposed on the side surfaces **18.** The second coating composition **22** is disposed on the portion of the first coating composition **20** that is disposed on the inner surface **16** and the side surfaces **18.**

### A. Suitable Stents

The stents that are particularly suitable for the present invention include any kind of stent for medical purposes which is known to the skilled artisan. Suitable stents include, for example, vascular stents such as self expanding stents and balloon expandable stents. Examples of self expanding stents useful in the present invention are illustrated in U.S. Patent Nos. 4,655,771 and 4,954,126 issued to Wallsten and 5,061,275 issued to Wallsten et al. Examples of appropriate balloon expandable stents are shown in U.S. Patent No. 5,449,373 issued to Pinchasik et al. In certain embodiments, the stent comprises a stent sidewall structure with openings therein. When such stents are used, it is in some instances preferable to have the coating disposed on the stent to conform to the stent to preserve the openings of the sidewall structure. In preferred embodiments, the stent suitable for the present invention is an Express stent. More preferably, the Express stent is an Express™ stent or an Express2™ stent (Boston Scientific, Inc. Natick, Ma.).

Stents that are suitable for the present invention may be fabricated from metallic, ceramic, or polymers, or a combination thereof. Preferably, the materials are biocompatible. Metallic material is more preferable. Suitable metallic materials include metals and alloys based on titanium (such as nitinol, nickel titanium alloys, thermo memory alloy materials), stainless steel, tantalum, nickel chrome, or certain cobalt alloys including cobalt chromium nickel alloys such as Elgiloy® and Phynox®. Metallic materials also include clad composite filaments, such as those disclosed in WO 94/16646.

Suitable ceramic materials include, but are not limited to, oxides, carbides, or nitrides of the transition elements such as titanium oxides, hafnium oxides, iridiumoxides, chromium oxides, aluminum oxides, and zirconiumoxides. Silicon based materials, such as silica, may also be used. The polymer may be biostable. Also, the polymer may be biodegradable. Suitable polymers include, but are not limited to, styrene isobutylene styrene, polyetheroxides, polyvinyl alcohol, polyglycolic acid, polylactic acid, polyamides, poly-2-hydroxy-butyrate, polycaprolactone, poly(lactic-co-clycolic)acid, and Teflon.

Polymers may be used for forming the stent in the present invention include without limitation isobutylene-based polymers, polystyrene-based polymers, polyacrylates, and polyacrylate derivatives, vinyl acetate-based polymers and its copolymers, polyurethane and its copolymers, silicone and its copolymers, ethylene vinyl-acetate, polyethylene terephtalate, thermoplastic elastomers, polyvinyl chloride, polyolefins, cellulosics, polyamides, polyesters, polysulfones, polytetrafluorethylenes, polycarbonates, acrylonitrile butadiene styrene copolymers, acrylics, polylactic acid, polyglycolic acid, polycaprolactone, polylactic acid-polyethylene oxide copolymers, cellulose, collagens, and chitins.

Other polymers that are useful as materials for stents include without limitation dacron polyester, poly(ethylene terephthalate), polycarbonate, polymethylmethacrylate, polypropylene, polyalkylene oxalates, polyvinylchloride, polyurethanes, polysiloxanes, nylons, poly(dimethyl siloxane), polycyanoacrylates, polyphosphazenes, poly(amino acids), ethylene glycol I dimethacrylate, poly(methyl methacrylate), poly(2-hydroxyethyl methacrylate), polytetrafluoroethylene poly(HEMA), polyhydroxyalkanoates, polytetrafluorethylene, polycarbonate, poly(glycolide-lactide) co-polymer, polylactic acid, poly(y-caprolactone), poly(γ-hydroxybutyrate), polydioxanone, poly(γ-ethyl glutamate), polyiminocarbonates, poly(ortho ester), polyanhydrides, alginate, dextran, chitin, cotton, polyglycolic acid, polyurethane, or derivatized versions thereof, i.e., polymers which have been modified to include, for example, attachment sites or cross-linking groups, *e.g*., RGD, in which the polymers retain their structural integrity while allowing for attachment of cells and molecules, such as proteins, nucleic acids, and the like.

Stents may also be made with non-polymers chemicals. Examples of useful non-polymers include sterols such as cholesterol, stigmasterol, β-sitosterol, and estradiol; cholesteryl esters such as cholesteryl stearate; C₁₂-C₂₄ fatty acids such as lauric acid, myristic acid, palmitic acid, stearic acid, arachidic acid, behenic acid, and lignoceric acid; C₁₈ -C₃₆ mono-, di- and triacylglycerides such as glyceryl monooleate, glyceryl monolinoleate, glyceryl monolaurate, glyceryl monodocosanoate, glyceryl monomyristate, glyceryl monodicenoate, glyceryl dipalmitate, glyceryl didocosanoate, glyceryl dimyristate, glyceryl didecenoate, glyceryl tridocosanoate, glyceryl trimyristate, glyceryl tridecenoate, glycerol tristearate and mixtures thereof; sucrose fatty acid esters such as sucrose distearate and sucrose palmitate; sorbitan fatty acid esters such as sorbitan monostearate, sorbitan monopalmitate and sorbitan tristearate; C₁₆ -C₁₈ fatty alcohols such as cetyl alcohol, myristyl alcohol, stearyl alcohol, and cetostearyl alcohol; esters of fatty alcohols and fatty acids such as cetyl palmitate and cetearyl palmitate; anhydrides of fatty acids such as stearic anhydride; phospholipids including phosphatidylcholine (lecithin), phosphatidylserine, phosphatidylethanolamine, phosphatidylinositol, and lysoderivatives thereof; sphingosine and derivatives thereof; sphingomyelins such as stearyl, palmitoyl, and tricosanyl sphingomyelins; ceramides such as stearyl and palmitoyl ceramides; glycosphingolipids; lanolin and lanolin alcohols; and combinations and mixtures thereof. Preferred non-polymers include cholesterol, glyceryl monostearate, glycerol tristearate, stearic acid, stearic anhydride, glyceryl monooleate, glyceryl monolinoleate, and acetylated monoglycerides.

### B. Suitable Therapeutic Agents

The term "therapeutic agent" encompasses biologically active material, and also genetic materials and biological materials. The therapeutic agents named herein include their analogs and derivatives. Non-limiting examples of suitable therapeutic agent include heparin, heparin derivatives, urokinase, dextrophenylalanine proline arginine chloromethylketone (PPack), enoxaprin, angiopeptin, hirudin, acetylsalicylic acid, tacrolimus, everolimus, rapamycin (sirolimus), pimecrolimus, amlodipine, doxazosin, glucocorticoids, betamethasone, dexamethasone, prednisolone, corticosterone, budesonide, sulfasalazine, rosiglitazone, mycophenolic acid, mesalamine, paclitaxel, 5-fluorouracil, cisplatin, vinblastine, vincristine, epothilones, methotrexate, azathioprine, adriamycin, mutamycin, endostatin, angiostatin, thymidine kinase inhibitors, cladribine, lidocaine, bupivacaine, ropivacaine, D-Phe-Pro-Arg chloromethyl ketone, platelet receptor antagonists, anti-thrombin antibodies, anti-platelet receptor antibodies, aspirin, dipyridamole, protamine, hirudin, prostaglandin inhibitors, platelet inhibitors, trapidil, liprostin, tick antiplatelet peptides, 5-azacytidine, vascular endothelial growth factors, growth factor receptors, transcriptional activators, translational promoters, antiproliferative agents, growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin, cholesterol lowering agents, vasodilating agents, agents which interfere with endogenous vasoactive mechanisms, antioxidants, probucol, antibiotic agents, penicillin, cefoxitin, oxacillin, tobranycin, angiogenic substances, fibroblast growth factors, estrogen, estradiol (E2), estriol (E3), 17-beta estradiol, digoxin, beta blockers, captopril, enalopril, statins, steroids, vitamins, paclitaxel (as well as its derivatives, analogs or paclitaxel bound to proteins, *e.g*. Abraxane™) 2'-succinyl-taxol, 2'-succinyl-taxol triethanolamine, 2'-glutaryl-taxol, 2'-glutaryl-taxol triethanolamine salt, 2'-O-ester with N-(dimethylaminoethyl) glutamine, 2'-O-ester with N-(dimethylaminoethyl) glutamide hydrochloride salt, nitroglycerin, nitrous oxides, nitric oxides, antibiotics, aspirins, digitalis, estrogen, estradiol and glycosides. In one embodiment, the therapeutic agent is a smooth muscle cell inhibitor or antibiotic. In another preferred embodiment, the therapeutic agent is paclitaxel or its analogs or derivatives (*i.e.* "paclitaxel"). In yet another preferred embodiment, the therapeutic agent is an antibiotic such as erythromycin, amphotericin, rapamycin, adriamycin, etc.

The term "genetic materials" means DNA or RNA, including, without limitation, of DNA/RNA encoding a useful protein stated below, intended to be inserted into a human body including viral vectors and non-viral vectors.

The term "biological materials" include cells, yeasts, bacteria, proteins, peptides, cytokines and hormones. Examples for peptides and proteins include vascular endothelial growth factor (VEGF), transforming growth factor (TGF), fibroblast growth factor (FGF), epidermal growth factor (EGF), cartilage growth factor (CGF), nerve growth factor (NGF), keratinocyte growth factor (KGF), skeletal growth factor (SGF), osteoblast-derived growth factor (BDGF), hepatocyte growth factor (HGF), insulin-like growth factor (IGF), cytokine growth factors (CGF), platelet-derived growth factor (PDGF), hypoxia inducible factor-1 (HIF-1), stem cell derived factor (SDF), stem cell factor (SCF), endothelial cell growth supplement (ECGS), granulocyte macrophage colony stimulating factor (GM-CSF), growth differentiation factor (GDF), integrin modulating factor (IMF), calmodulin (CaM), thymidine kinase (TK), tumor necrosis factor (TNF), growth hormone (GH), bone morphogenic protein (BMP) (*e.g.*, BMP-2, BMP-3, BMP-4, BMP-5. BMP-6 (Vgr-1), BMP-7 (PO-1), BMP-8, BMP-9, BMP-10, BMP-11, BMP-12, BMP-14, BMP-15, BMP-16, etc.), matrix metalloproteinase (MMP), tissue inhibitor of matrix metalloproteinase (TIMP), cytokines, interleukin (*e.g*., IL-1, IL-2, IL-3, IL-4, IL-5, IL-6, IL-7, IL-8, IL-9, IL-10, IL-11, IL-12, IL-15, etc.), lymphokines, interferon, integrin, collagen (all types), elastin, fibrillins, fibronectin, vitronectin, laminin, glycosaminoglycans, proteoglycans, transferrin, cytotactin, cell binding domains (*e.g*., RGD), and tenascin. Currently preferred BMP's are BMP-2, BMP-3, BMP-4, BMP-5, BMP-6, BMP-7. These dimeric proteins can be provided as homodimers, heterodimers, or combinations thereof, alone or together with other molecules. Cells can be of human origin (autologous or allogeneic) or from an animal source (xenogeneic), genetically engineered, if desired, to deliver proteins of interest at the transplant site. The delivery media can be formulated as needed to maintain cell function and viability. Cells include progenitor cells (*e.g*., endothelial progenitor cells), stem cells (*e.g*., mesenchymal, hematopoietic, neuronal), stromal cells, parenchymal cells, undifferentiated cells, fibroblasts, macrophage, and satellite cells.

Other non-genetic therapeutic agents include:
- anti-thrombogenic agents such as heparin, heparin derivatives, urokinase, and PPack (dextrophenylalanine proline arginine chloromethylketone);
- anti-proliferative agents such as enoxaprin, angiopeptin, or monoclonal antibodies capable of blocking smooth muscle cell proliferation, hirudin, acetylsalicylic acid, tacrolimus, everolimus, amlodipine and doxazosin;
- anti-inflammatory agents such as glucocorticoids, betamethasone, dexamethasone, prednisolone, corticosterone, budesonide, estrogen, sulfasalazine, rosiglitazone, mycophenolic acid and mesalamine;
- anti-neoplastic/anti-proliferative/anti-miotic agents such as paclitaxel, 5-fluorouracil, cisplatin, vinblastine, vincristine, epothilones, methotrexate, azathioprine, adriamycin and mutamycin; endostatin, angiostatin and thymidine kinase inhibitors, cladribine, taxol and its analogs or derivatives;
- anesthetic agents such as lidocaine, bupivacaine, and ropivacaine;
- anti-coagulants such as D-Phe-Pro-Arg chloromethyl ketone, an RGD peptide-containing compound, heparin, antithrombin compounds, platelet receptor antagonists, anti-thrombin antibodies, anti-platelet receptor antibodies, aspirin (aspirin is also classified as an analgesic, antipyretic and anti-inflammatory drug), dipyridamole, protamine, hirudin, prostaglandin inhibitors, platelet inhibitors, antiplatelet agents such as trapidil or liprostin and tick antiplatelet peptides;
- DNA demethylating drugs such as 5-azacytidine, which is also categorized as a RNA or DNA metabolite that inhibit cell growth and induce apoptosis in certain cancer cells;
- vascular cell growth promoters such as growth factors, vascular endothelial growth factors (VEGF, all types including VEGF-2), growth factor receptors, transcriptional activators, and translational promoters;
- vascular cell growth inhibitors such as anti-proliferative agents, growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin;
- cholesterol-lowering agents, vasodilating agents, and agents which interfere with endogenous vasoactive mechanisms;
- anti-oxicianis, such as probucoi;
- antibiotic agents, such as penicillin, cefoxitin, oxacillin, tobranycin, rapamycin (sirolimus);
- angiogenic substances, such as acidic and basic fibroblast growth factors, estrogen including estradiol (E2), estriol (E3) and 17-beta estradiol;
- drugs for heart failure, such as digoxin, beta-blockers, angiotensin-converting enzyme (ACE) inhibitors including captopril and enalopril, statins and related compounds; and
- macrolide agents such as sirolimus, pimerolimus, or everolimus.

Preferred biological materials include anti-proliferative drugs such as steroids, vitamins, and restenosis-inhibiting agents. Preferred restenosis-inhibiting agents include microtubule stabilizing agents such as Taxol®, paclitaxel (*i.e*., paclitaxel, paclitaxel analogs, or paclitaxel derivatives, and mixtures thereof). For example, derivatives suitable for use in the present invention include 2'-succinyl-taxol, 2'-succinyl-taxol triethanolamine, 2'-glutaryl-taxol, 2'-glutaryl-taxol triethanolamine salt, 2'-O-ester with N-(dimethylaminoethyl) glutamine, and 2'-O-ester with N-(dimethylaminoethyl) glutamide hydrochloride salt.

Other suitable therapeutic agents include tacrolimus; halofuginone; inhibitors of HSP90 heat shock proteins such as geldanamycin; microtubule stabilizing agents such as epothilone D; phosphodiesterase inhibitors such as cliostazole; Barkct inhibitors; phospholamban inhibitors; and Serca 2 gene/pmteins.

Other preferred therapeutic agents include nitroglycerin, nitrous oxides, nitric oxides, aspirins, digitalis, estrogen derivatives such as estradiol and glycosides.

In one embodiment, the therapeutic agent is capable of altering the cellular metabolism or inhibiting a cell activity, such as protein synthesis, DNA synthesis, spindle fiber formation, cellular proliferation, cell migration, microtubule formation, microfilament formation, extracellular matrix synthesis, extracellular matrix secretion, or increase in cell volume. In another embodiment, the therapeutic agent is capable of inhibiting cell proliferation and/or migration.

In certain embodiments, the therapeutic agents for use in the medical devices of the present invention can be synthesized by methods well known to one skilled in the art. Alternatively, the therapeutic agents can be purchased from chemical and pharmaceutical companies.

Methods suitable for applying therapeutic agents to the devices of the present invention preferably do not alter or adversely impact the therapeutic properties of the therapeutic agent.

### C. Suitable Polymers

Polymers useful for forming the coatings should be ones that are biocompatible, particularly during insertion or implantation of the device into the body and avoids irritation to body tissue. Examples of such polymers include, but not limited to, polyurethanes, polyisobutylene and its copolymers, silicones, and polyesters. Other suitable polymers include polyolefins, polyisobutylene, ethylene-alphaolefin copolymers, acrylic polymers and copolymers, vinyl halide polymers and copolymers such as polyvinyl chloride, polyvinyl ethers such as polyvinyl methyl ether, polyvinylidene halides such as polyvinylidene fluoride and polyvinylidene chloride, polyacrylonitrile, polyvinyl ketones, polyvinyl aromatics such as polystyrene, polyvinyl esters such as polyvinyl acetate; copolymers of vinyl monomers, copolymers of vinyl monomers and olefins such as ethylene-methyl methacrylate copolymers, acrylonitrile-styrene copolymers, ABS resins, ethylene-vinyl acetate copolymers, polyamides such as Nylon 66 and polycaprolactone, alkyd resins, polycarbonates, polyoxyethylenes, polyimides, polyethers, epoxy resins, polyurethanes, rayon-triacetate, cellulose, cellulose acetate, cellulose butyrate, cellulose acetate butyrate, cellophane, cellulose nitrate, cellulose propionate, cellulose ethers, carboxymethyl cellulose, collagens, chatins, polylactic acid, polyglycolic acid, polylactic acid-polyethylene oxide copolymers, of styrene and isobutylene copolymers.

When the polymer is being applied to a part of the medical device, such as a stent, which undergoes mechanical challenges, e.g. expansion and contraction, the polymers are preferably selected from elastomeric polymers such as silicones (e.g. polysiloxanes and substituted polysiloxanes), polyurethanes, thermoplastic elastomers, ethylene vinyl acetate copolymers, polyolefin elastomers, and EPD rubbers. The polymer is selected to allow the coating to better adhere to the surface of the strut when the stent is subjected to forces or stress. Furthermore, although the coating can be formed by using a single type of polymer, various combinations of polymers can be employed.

Generally, when a hydrophilic therapeutic agent is used then a hydrophilic polymer having a greater affinity for the therapeutic agent than another material that is less hydrophilic is preferred. When a hydrophobic therapeutic agent is used then a hydrophobic polymer having a greater affinity for the therapeutic agent is preferred.

Examples of suitable hydrophobic polymers or monomers include, but not limited to, polyolefins, such as polyethylene, polypropylene, poly(1-butene), poly(2-butene), poly(1-pentene), poly(2-pentene), poly(3-methyl-1-pentene), poly(4-methyl-1-pentene), poly(isoprene), poly(4-methyl-1-pentene), ethylene-propylene copolymers, ethylene-propylene-hexadiene copolymers, ethylene-vinyl acetate copolymers, blends of two or more polyolefins and random and block copolymers prepared from two or more different unsaturated monomers; styrene polymers, such as poly(styrene), poly(2-methylstyrene), styrene-acrylonitrile copolymers having less than about 20 mole-percent acrylonitrile, and styrene-2,2,3,3,-tetrafluoropropyl methacrylate copolymers; halogenated hydrocarbon polymers, such as poly(chlorotrifluoroethylene), chlorotrifluoroethylene-tetrafluoroethylene copolymers, poly(hexafluoropropylene), poly(tetrafluoroethylene), tetrafluoroethylene, tetrafluoroethylene-ethylene copolymers, poly(trifluoroethylene), poly(vinyl fluoride), and poly(vinylidene fluoride); vinyl polymers, such as poly(vinyl butyrate), poly(vinyl decanoate), poly(vinyl dodecanoate), poly(vinyl hexadecanoate), poly(vinyl hexanoate), poly(vinyl propionate), poly(vinyl octanoate), poly(heptafluoroisopropoxyethylene), poly(heptafluoroisopmpoxypropylene), and poly(methacrylonitrile); acrylic polymers, such as poly(n-butyl acetate), poly(ethyl acrylate), poly(1-chlorodifluoromethyl)tetrafluoroethyl acrylate, poly di(chlorofluoromethyl)fluoromethyl acrylate, poly(1,1-dihydroheptafluorobutyl acrylate), poly(1,1-dihydropentafluoroisopropyl acrylate), poly(1,1-dihydropentadecafluorooctyl acrylate), poly(heptafluoroisopropyl acrylate), poly 5-(heptafluoroisopropoxy)pentyl acrylate, poly 11-(heptafluoroisopropoxy)undecyl acrylate, poly 2-(heptafluoropropoxy)ethyl acrylate, and poly(nonafluoroisobutyl acrylate); methacrylic polymers, such as poly(benzyl methacrylate), poly(n-butyl methacrylate), poly(isobutyl methacrylate), poly(t-butyl methacrylate), poly(t-butylaminoethyl methacrylate), poly(dodecyl methacrylate), poly(ethyl methacrylate), poly(2-ethylhexyl methacrylate), poly(n-hexyl methacrylate), poly(phenyl methacrylate), poly(n-propyl methacrylate), poly(octadecyl methacrylate), poly(1,1-dihydropentadecafluorooctyl methacrylate), poly(heptafluoroisopropyl methacrylate), poly(heptadecafluorooctyl methacrylate), poly(1-hydrotetrafluoroethyl methacrylate), poly(1,1-dihydrotetrafluoropropyl methacrylate), poly(1-hydrohexafluoroisopropyl methacrylate), and poly(t-nonafluorobutyl methacrylate); polyesters, such a poly(ethylene terephthalate) and poly(butylene terephthalate); condensation type polymers such as and polyurethanes and siloxane-urethane copolymers; polyorganosiloxanes, i.e., polymeric materials characterized by repeating siloxane groups, represented by Ra SiO 4-a/2, where R is a monovalent substituted or unsubstituted hydrocarbon radical and the value of a is 1 or 2; and naturally occurring hydrophobic polymers such as rubber.

Examples of suitable hydrophilic polymers or monomers include, but not limited to; (meth)acrylic acid, or alkaline metal or ammonium salts thereof; (meth)acrylamide; (meth)acrylonitrile; those polymers to which unsaturated dibasic, such as maleic acid and fumaric acid or half esters of these unsaturated dibasic acids, or alkaline metal or ammonium salts of these dibasic adds or half esters, is added; those polymers to which unsaturated sulfonic, such as 2-acrylamido-2-methylpropanesulfonic, 2-(meth)acryloyledianesulfonic acid, or alkaline metal or ammonium salts thereof, is added; and 2-hydroxyethyl (meth)acrylate and 2-hydroxypropyl (meth)acrylate.

Polyvinyl alcohol is also an example of hydrophilic polymer. Polyvinyl alcohol may contain a plurality of hydrophilic groups such as hydroxyl, amido, carboxyl, amino, ammonium or sulfonyl (-SO₃). Hydrophilic polymers also include, but are not limited to, starch, polysaccharides and related cellulosic polymers; polyalkylene glycols and oxides such as the polyethylene oxides; polymerized ethylenically unsaturated carboxylic acids such as acrylic, mathacrylic and maleic acids and partial esters derived from these acids and polyhydric alcohols such as the alkylene glycols; homopolymers and copolymers derived from acrylamide; and homopolymers and copolymers of vinylpyrrolidone.

Other suitable polymers include without limitation: polyurethanes, silicones (*e.g.*, polysiloxanes and substituted polysiloxanes), and polyesters, styrene-isobutylene-copolymers. Other polymers which can be used include ones that can be dissolved and cured or polymerized on the medical device or polymers having relatively low melting points that can be blended with therapeutic agents. Additional suitable polymers include, but are not limited to, thermoplastic elastomers in general, polyolefins, polyisobutylene, ethylene-alphaolefin copolymers, acrylic polymers and copolymers, vinyl halide polymers and copolymers such as polyvinyl chloride, polyvinyl ethers such as polyvinyl methyl ether, polyvinylidene halides such as polyvinylidene fluoride and polyvinylidene chloride, polyacrylonitrile, polyvinyl ketones, polyvinyl aromatics such as polystyrene, polyvinyl esters such as polyvinyl acetate, copolymers of vinyl monomers, copolymers of vinyl monomers and olefins such as ethylene-methyl methacrylate copolymers, acrylonitrile-styrene copolymers, ABS (acrylonitrile-butadiene-styrene) resins, ethylene-vinyl acetate copolymers, polyamides such as Nylon 66 and polycaprolactone, alkyd resins, polycarbonates, polyoxymethylenes, polyimides, polyethers, polyether block amides, epoxy resins, rayon-triacetate, cellulose, cellulose acetate, cellulose butyrate, cellulose acetate butyrate, cellophane, cellulose nitrate, cellulose propionate, cellulose ethers, carboxymethyl cellulose, collagens, chitins, polylactic acid, polyglycolic acid, polylactic acid-polyethylene oxide copolymers, EPD (ethylene-propylene-diene) rubbers, fluoropolymers, fluorosilicones, polyethylene glycol, polysaccharides, phospholipids, and combinations of the foregoing.

### D. Methods for Forming the Coatings

The coating compositions can be prepared by dissolving or suspending a polymer and/or therapeutic agent in a solvent. Solvents that may be used to prepare coating compositions include ones which can dissolve or suspend the polymer and/or therapeutic agent in solution. Examples of suitable solvents include, but are not limited to, tetrahydrofuran, methylethylketone, chloroform, toluene, acetone, isooctane, 1,1,1, trichloroethane, dichloromethane, isopropanol, IPA, and mixture thereof.

The aforementioned coated medical devices can be made by applying coating compositions onto the surface of the medical device. Coating compositions can be applied by any method to a surface of a medical device or to another coating composition known by one skilled in the art. The different surfaces may be coated by the same or different methods. Suitable methods for applying the coating compositions to the medical devices include, but are not limited to, spray-coating, painting, rolling, electrostatic deposition, ink jet coating, dip coating, spin coating and a batch process such as air suspension, pan-coating or ultrasonic mist spraying, or a combination thereof.

In embodiments where a coating composition is to be applied to fewer than all the surfaces of the struts of a stent, such as on the stents described above, it is preferable to employ coating methods that selectively apply the coating composition. For instance, a first coating composition can be deposited onto a substrate. The substrate is preferably made from materials that has minimal adhesion the coating composition so that the coating composition can be easily removed and transferred to the surface. Then, the outer surface of the struts may be rolled over the coated substrate to transfer the coating composition to the outer surfaces of the struts.

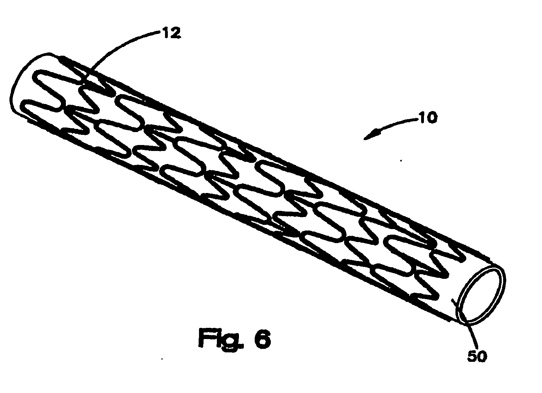

Also, it may be preferable to mask or cover the surface that is not to be coated with a particular coating composition. For instance in the embodiment in **Figure 2****,** to avoid having the first coating composition **20** disposed upon the inner surface **16** of the strut **12** and the side surfaces **18** of the strut **12,** these surfaces can be masked. In one embodiment, the inner surface **16** can be masked by placing the stent **10** on a mandrel **50,** such as that shown in **Figure 6****.** The inner surface **16** which is placed against the mandrel will not be exposed to a coating composition that is applied to the outer surface **14**. For example, in one embodiment, the stent that is mounted on the mandrel may then be rolled over a substrate containing a coating composition to transfer the coating composition to the outer surface of the struts to form the embodiment in **Figure 2****.** Alternatively, the stent **10** can be placed on the mandrel **50** and the outer and side surfaces of the strut are spray-coating with the first composition **20** so that the embodiment of **Figure 2A** is formed.

In an alternative embodiment, a bare stent can be dip coated with a material such as wax. In order to selectively coat particular portions of the stent, the wax coating can be ground off in selected locations, exposing the chosen locations of the stent struts. Subsequently, the stent can be spray coated, dipped, painted, rolled or by other means coated on the exposed locations. After the coating is complete, the wax on the remaining portions of the stent can be removed.

In embodiments where the coating composition is to be applied to the inner or side surfaces, the outer surface can be masked. For example, in the embodiments shown in **Figures 3-3C****,** the outer surface is masked when the second coating composition is applied to the inner surface 16 and/or side surfaces **18.** The outer surface **14** can be masked, for instance, by application of a protective wrap to that surface. The protective wrap is a material that would protect the coated surface from exposure to the coating applied to the opposing surface. Suitable material for this protective wrap include, for example, PTFE film, dyna-leap, Kapton®, or any other appropriate type of covering or wrapping material. The protective wrap preferably extends for the length of the stent, and is secured so that it does not unwrap. The protective wrap serves to protect the outer surface **14** from exposure to the second coating **22** composition as it is being applied to the inner surface **16.** Thus, the protective wrap will protect an outer surface **14** that has been already coated from additional deposition of the coating to be applied to the inner surfaces **16** and side surfaces **18.** After the inner surfaces **16** and side surfaces **18** of the struts **12** of the medical device have been coated, the wrap covering the outer surface **14** may be removed. A wrap can also be used to cover other surfaces, such as the inner and side surface, to prevent a coating composition from being disposed on such surfaces.

In embodiments, where the inner surface **16** and side surfaces **18** of stent **10** are to be coated, it may be preferable to use a spraying process. For example, a nozzle assembly may be used to spray a coating composition onto the inner surface. The nozzle assembly may be in the form of a cone that sprays the coating composition at an angle. The angle of the spray from the nozzles may need to be adjusted to ensure uniform thickness of the coating on the inner surface. Also, a nozzle assembly with small spray nozzles can be inserted into one end of the stent and moved through the stent until it extends past the opposite end of the stent. Preferably, the spray mist flow is started while the nozzle is still outside of the stent. This step places a coating composition on the inside surface and one side surface of the struts of the stent. The coating process may be repeated again. Preferably, the spray nozzle is inserted into the other end of the stent to coat the other side surface of the struts. By repeating the spraying from two directions, both side surfaces are coated with a coating composition.

Masking and selective coating techniques can be used to form the embodiments shown in the figures. For example, in the embodiments shown in **Figures 3 and 3A****,** the side surfaces **18** of the strut **12** are masked and the first coating composition **20** is applied to the inner **16** and outer **14** surfaces. Thereafter, masking may be used to selectively dispose the second coating composition **22** on just the first coating composition **20** disposed on the inner surface **16** (as in **Figure 3****)** or on the side surfaces **18** and the first coating composition **20** disposed on the inner surface **16** (as in **Figure 3A****).** In the embodiments in **Figures 3B-3C****,** the first coating composition **20** is applied to the outer **14,** inner **16** and side **18** surfaces. Thereafter, masking may be used to selectively dispose the second coating composition **22** on just the first coating composition **20** disposed on the inner surface **16** (as in **Figure 3B**) or on the first coating composition **20** disposed on the inner surface **16** and side surfaces **18** (as in **Figure 3C**).

After a coating composition has been applied, it can be cured. Curing is defined as the process of converting the polymeric material into the finished or useful state by the application of heat, vacuum, and/or chemical agents which induce physico-chemical changes. The applicable time and temperature for curing are determined by the particular polymer involved and particular therapeutic agent used, if any, as known by one skilled in the art. The coated medical devices may thereafter be subjected to a post-cure process wherein the medical devices are exposed to a low energy for stabilization of the coating. Also, after the medical device is coated, it preferably should be sterilized by methods of sterilization as known in the art.
In use, a coated medical device, such as an expandable stent, according to the present invention can be made to provide desired release profile of the therapeutic agent. The medical devices and stents of the present invention may be used for any appropriate medical procedure. Delivery of the medical device can be accomplished using methods well known to those skilled in the art, such as mounting the stent on an inflatable balloon disposed at the distal end of a delivery catheter.

## Claims

1. An intravascular balloon-expandable stent comprising:
a metal stent sidewall structure designed for implantation into a blood vessel of a patient, wherein the sidewall structure comprises a plurality of struts and openings in the sidewall structure, and wherein the struts each have an outer surface and an inner surface opposite the outer surface and side surfaces adjacent to the outer surface and
inner surface; **characterized by**
a first coating composition disposed on the outer surface, inner surface and side surfaces of the struts, wherein the first coating composition comprises a therapeutic agent and a first biostable polymer; a second coating composition disposed on the first coating composition that is disposed on the inner surface and side surfaces of the struts, wherein the second coating composition comprises a second biostable polymer that has less tackiness than the first polymer and is free of any therapeutic agent when applied to the first coating composition that is disposed on the inner surface and the side surfaces; and wherein the second coating composition is not disposed on the first coating composition that is disposed on the outer surface of the struts.

2. The stent of claim 1, wherein the therapeutic agent comprises an anti-thrombogenic agent, anti-angiogenesis agent, anti-proliferative agent, anti-restensosis agent, growth factor, radiochemical or antibiotic.

3. The stent of claim 1, wherein the therapeutic agent comprises paclitaxel, sirolimus, everolimus, tacrolimus, or pimecrolimus.

4. The stent of claim 1, wherein the first coating composition conforms to the outer surface and inner surface of the at least one strut to preserve the openings of the sidewall structure and the second coating composition conforms to the inner surface of the at least one strut to preserve the openings of the sidewall structure.

5. The stent of claim 1, wherein the second polymer is harder than the first polymer.

6. The stent of claim 5, wherein second polymer has a hardness of at least 40 A.

7. The stent of claim 1, wherein the second polymer has a tackiness of less than 50 g.

8. The stent of claim 7, wherein the second polymer has a tackiness of 3 g to about 30 g.

9. The stent of claim 1, wherein the second polymer comprises polyamide.

## Patentansprüche

1. Intravasculärer, Ballon-expandierbarer Stent, umfassend:
eine Metallstent-Seitenwandstruktur, konstruiert zur Implantation in ein Blutgefäß eines Patienten, wobei die Seitenwandstruktur eine Vielzahl von Streben und Öffnungen in der Seitenwandstruktur umfasst und wobei die Streben jeweils eine äußere Oberfläche und eine innere Oberfläche entgegengesetzt zu der äußeren Oberfläche sowie Seitenoberflächen angrenzend an die äußere und die innere Oberfläche aufweisen; **gekennzeichnet durch**
eine erste Beschichtungszusammensetzung, angeordnet auf der äußeren Oberfläche, inneren Oberfläche und den Seitenoberflächen der Streben,
wobei die erste Beschichtungszusammensetzung ein therapeutisches Mittel und ein erstes biostabiles Polymer umfasst; eine zweite Beschichtungszusammensetzung, angeordnet auf der ersten Beschichtungszusammensetzung, welche auf der inneren Oberfläche und den Seitenoberflächen der Streben angeordnet ist, wobei die zweite Beschichtungszusammensetzung ein zweites biostabiles Polymer umfasst,
welches weniger Haftvermögen aufweist als das erste Polymer und von jeglichem therapeutischen Mittel frei ist, wenn sie auf die erste Beschichtungszusammensetzung aufgebracht wird, welche auf der inneren Oberfläche und den Seitenoberflächen der Streben angeordnet ist; und wobei die zweite Beschichtungszusammensetzung nicht auf der ersten Beschichtungszusammensetzung angeordnet ist, welche auf der äußeren Oberfläche der Streben angeordnet ist.

2. Stent gemäß Anspruch 1, wobei das therapeutische Mittel ein Antithrombosemittel, Antiangiogenesemittel, Antiproliferationsmittel, Antirestenosemittel, einen Wachstumsfaktor, eine Radiochemikalie oder ein Antibiotikum umfasst.

3. Stent gemäß Anspruch 1, wobei das therapeutische Mittel Paclitaxel, Sirolimus, Everolimus, Tacrolimus oder Pimecrolius umfasst.

4. Stent gemäß Anspruch 1, wobei die erste Beschichtungszusammensetzung sich an die äußere Oberfläche und die innere Oberfläche der mindestens einen Strebe anpasst, um die Öffnungen der Seitenwandstruktur beizubehalten, und die zweite Beschichtungszusammensetzung sich an die innere Oberfläche der mindestens einen Strebe anpasst, um die Öffnungen der Seitenwandstruktur beizubehalten.

5. Stent gemäß Anspruch 1, wobei das zweite Polymer härter als das erste Polymer ist.

6. Stent gemäß Anspruch 5, wobei das zweite Polymer eine Härte von mindestens 40 A hat.

7. Stent gemäß Anspruch 1, wobei das zweite Polymer ein Haftvermögen von weniger als 50g hat.

8. Stent gemäß Anspruch 7, wobei das zweite Polymer ein Haftvermögen von 3g bis etwa 30g hat.

9. Stent gemäß Anspruch 1, wobei das zweite Polymer Polyamid umfasst.

## Revendications

1. Stent intravasculaire dilatable par ballonnet, comprenant :
un structure de paroi latérale de Stent métallique, construite pour l'implantation dans un vaisseau de sang d'un patient, dans laquelle la structure de paroi latérale comprend une multitude d'étais et ouvertures dans la structure de paroi latérale et dans laquelle les étais ont chacune une surface extérieure et une surface intérieure vis-à-vis de la surface extérieure et des surfaces latérales adjacentes aux surfaces extérieure et intérieure; **caractérisé par**
une première composition de couchage, disposée sur la surface extérieure, la surface intérieure et les surfaces latérales des étais, dans laquelle la première composition de couchage comprend un agent thérapeutique et un premier polymère biostable; une deuxième composition de couchage, disposée sur la première composition de couchage qui est disposée sur la surface intérieure et les surfaces latérales des étais, la deuxième composition de couchage comprenant un deuxième polymère biostable qui a moins d'adhérence que le premier polymère et est libre d'aucun agent thérapeutique quand elle est appliquée sur la première composition de couchage qui est elle-même disposée sur la surface intérieure et les surfaces latérales; et dans laquelle la deuxième composition de couchage n'est pas disposée sur la première composition de couchage qui est disposée sur la surface extérieure des étais.

2. Stent selon la revendication 1, dans lequel l'agent thérapeutique comprend un agent anti-thrombose, anti-angiogenèse, anti-prolifération, anti-resténose, un facteur de croissance, un agent radiochimique ou un antibiotique.

3. Stent selon la revendication 1, dans lequel l'agent thérapeutique comprend du paclitaxel, sirolimus, évérolimus, tacrolimus ou pimécrolius.

4. Stent selon la revendication 1, dans lequel la première composition de couchage se conforme à la surface extérieure et la surface intérieure de l'au moins un étai, afin de préserver les ouvertures de la structure de paroi latérale, et la deuxième composition de couchage se conforme à la surface intérieure de l'au moins un étai, afin de préserver les ouvertures de la structure de paroi latérale.

5. Stent selon la revendication 1, dans lequel le deuxième polymère est plus dur que le premier polymère.

6. Stent selon la revendication 5, dans lequel le deuxième polymère a une dureté d'au moins 40 A.

7. Stent selon la revendication 1, dans lequel le deuxième polymère a un degré d'adhérence de moins que 50 g.

8. Stent selon la revendication 7, dans lequel le deuxième polymère a un degré d'adhérence de 3g à environ 30g.

9. Stent selon la revendication 1, dans lequel le deuxième polymère comprend du polyamide.
